# EUROPEAN PATENT SPECIFICATION

(11) **EP 3 781 029 B1**
(45) Date of publication and mention of the grant of the patent: **05.04.2023**
(21) Application number: 19715498.2
(22) Date of filing: 10.04.2019
(51) Int. Cl.: A61B 5/021, A61B 5/024

(54) **DEVICE, SYSTEM AND METHOD FOR SUPPORTING DETECTION OF RETURN OF SPONTANEOUS CIRCULATION DURING CARDIOPULMONARY RESUSCITATION**
VORRICHTUNG, SYSTEM UND VERFAHREN ZUR UNTERSTÜTZUNG DER DETEKTION DER RÜCKKEHR VON SPONTANER ZIRKULATION WÄHREND KARDIOPULMONALER WIEDERBELEBUNG
DISPOSITIF, SYSTÈME ET PROCÉDÉ POUR SOUTENIR LA DÉTECTION DU RETOUR DE LA CIRCULATION SPONTANÉE PENDANT LA RÉANIMATION CARDIORESPIRATOIRE

(30) Priority: 17.04.2018 EP 18167635
(43) Date of publication of application: 24.02.2021
(73) Proprietor: Koninklijke Philips N.V., 5656 AG Eindhoven (NL)
(72) Inventor: WIJSHOFF, Ralph, Wilhelm, Christianus, Gemma, Rosa, 5656 AE Eindhoven (NL); WOERLEE, Pierre, Hermanus, 5656 AE Eindhoven (NL); MUEHLSTEFF, Jens, 5656 AE Eindhoven (NL)
(74) Representative: Philips Intellectual Property & Standards
(86) International application number: PCT/EP2019/059015
(87) International publication number: WO 2019/201689

(56) References cited:
- WO-A1-2017/072055
- US-A1- 2007 191 688
- US-A1- 2014 323 846
- US-A1- 2016 157 739

## Description

### FIELD OF THE INVENTION

The present invention relates to a device providing quantitative support for detection of return of spontaneous circulation during cardiopulmonary resuscitation. The present invention further relates to a system incorporating the aforementioned device and a corresponding method.

### BACKGROUND OF THE INVENTION

Detecting return of spontaneous circulation (ROSC) during cardiopulmonary resuscitation (CPR) is challenging. High-quality cardiopulmonary resuscitation requires minimizing interruptions of chest compressions. Typically, during CPR, chest compressions are interrupted to perform pulse checks, mostly by manual palpation for arterial pulsations. Futile pulse checks lead to unnecessary interruptions in chest compressions. Moreover, pulse checks by manual palpation are known to be time-consuming, especially when a spontaneous pulse is absent. This can lead to prolonged interruptions in compressions, which in turn lead to a degrading quality of the delivered CPR. In fact, interruptions in chest compressions reduce blood flow and thereby the chance of achieving ROSC. Moreover, manual palpation is known to be unreliable, even when performed by expert clinicians.

Furthermore, it is known that delivering compressions on a beating heart may lead to re-arrest. Therefore, it is important to know in time when the heart is sufficiently strong and no longer needs additional support, so compressions can be stopped.

Alternatively, a reliable and objective measurement to support ROSC detection is an invasive arterial blood pressure measurement, which can be interpreted to indicate ROSC when the systolic blood pressure (SBP) is higher than, e.g., 60 mmHg. Also monitoring of end-tidal CO2 or central venous oxygen saturation may be considered for ROSC detection. However, these measurements require placement of catheters or a secured airway. Hence, they are invasive measurements and are consequently typically neither desired nor available.

Trans-thoracic impedance (TTI) measurements and near-infrared spectroscopy (NIRS) are non-invasive methods that can provide pulse detection support during CPR, but TTI is strongly influenced by chest compressions and NIRS responds slowly upon ROSC.

US 2007/191688 A1 discloses embodiments related to a system and method for detecting a response of a patient to an external stimulus. An exemplary method comprises the acts of generating at least one time series of at least one physiologic parameter, detecting the external stimulus, detecting at least one response associated with the stimulus, the response comprising at least one of a pattern and a value, and outputting an indication of the response.

US 2014/323846 A1 discloses a system for determining a hemodynamic status of an individual may include a photoplethysmography (PPG) sub-system configured to detect a PPG signal and a response triggering module configured to analyze the PPG signal and output one or more response triggers based on a changing feature of the PPG signal within a time window. Each of the one or more response triggers may relate to an instruction to initiate detection of at least one physiological characteristic of the individual. A blood pressure (BP) variability index determination module is configured to determine a BP variability index related to a hemodynamic status of the individual based on a frequency or pattern of the one or more response triggers.

### SUMMARY OF THE INVENTION

It is an object of the present invention to provide a device for supporting detection of return of spontaneous circulation during cardiopulmonary resuscitation which works non-invasively and can prevent unnecessary interruptions in chest compressions.

In a first aspect of the present invention there is provided a device for supporting detection of return of spontaneous circulation during cardiopulmonary resuscitation, the device comprising
- a PPG input configured to obtain one or more photoplethysmography, PPG, signals of a patient;
- a BP output configured to provide a BP control signal for controlling a BP sensor to start measuring blood pressure, BP, of the patient;
- a BP input configured to obtain one or more BP measurements;and
- a processing unit configured to analyze one or more predefined PPG signal properties of the one or more PPG signals and to generate the BP control signal if one or more of the PPG signal properties fulfill a predefined condition, wherein the one or more PPG signal properties correspond to at least pulse rate, pulse strength, and duration of the pulse presence of the patient, and wherein the predefined condition comprises respective thresholds for pulse rate, pulse strength, and pulse presence duration, and wherein the predefined condition is for detecting return of a spontaneous pulse;wherein the processing unit is configured to assess a status of an indicator of cardiogenic output indicating return of spontaneous circulation based on a combination of the one or more PPG signals with the BP measurements.

In a second aspect the present invention provides a system for supporting detection of return of spontaneous circulation during cardiopulmonary resuscitation, the system comprising
- one or more PPG sensors configured to measure one or more PPG signals;
- the device according to the first aspect configured to generate a BP control signal by use of the one or more PPG signals; and
- a BP sensor configured to perform BP measurements based on the BP control signal; wherein the device is configured to assess a status of an indicator of cardiogenic output indicating return of spontaneous circulation based on a combination of the one or more PPG signals with the BP measurements.

In yet further aspects of the present invention, there are provided a corresponding method, a computer program which comprises program code means for causing a computer to perform the steps of the method disclosed herein when said computer program is carried out on a computer.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the idea to provide a device for guiding a user, particularly a clinician, through CPR with a PPG-triggered BP measurement indicating return of spontaneous circulation. This may prevent unnecessary interruptions during compressions as well as delivering compressions on a beating heart for too long, which may cause re-arrest. Furthermore, the present invention may support determining when to administer vasopressors. The present invention is particularly intended to improve in-hospital CPR and out-of hospital CPR by medical professionals. The present invention may also be used in public AEDs with PPG and blood measurements on board.

Currently, there is no non-invasive, blood-pressure related, quantitative indicator of ROSC.

PPG can non-invasively detect presence or absence of a spontaneous pulse during CPR. Photoplethysmography is an optical measurement technique that evaluates a time-variant change of light reflectance or transmission of an area or volume of interest. PPG is based on the principle that blood absorbs light more than surrounding tissue, so variations in blood volume with every heart beat affect transmission or reflectance correspondingly. PPG measurements can be carried out non-invasively at the skin's surface, where a light source and detector being in contact with the patient's skin form the PPG sensor of the system. However, PPG measurements can also be carried out by a PPG sensor being arranged at a distance from the tissue, where the light source and/or detector are not in contact with the tissue, such as in the case of camera-based measurements.

After a defibrillation shock, presence of spontaneous pulses can be observed in the PPG signal during few-second pauses in the compressions and during ongoing compressions. During ongoing compressions, an increase in the complexity of the PPG waveform indicates spontaneous cardiac activity underlying the chest compressions. Furthermore, a decrease in the baseline of the PPG signal may also be considered an indicator of cardiogenic output, i.e. presence of heart beats or viability of the heart. Moreover, spectral analysis of the PPG signal during ongoing compressions can show presence of a second harmonic series, where a first harmonics series in the PPG signal spectrum results from chest compression and the second harmonic series in the PPG signal spectrum results from spontaneous cardiac activity. Presence of a spontaneous pulse during CPR may be confirmed by an increase in a simultaneously measured invasive arterial blood pressure (iABP).

However, PPG does not provide a quantitative measure of the status of the circulatory system. In fact, PPG may detect presence of spontaneous pulses at sub-life-supporting blood pressures. PPG does not give direct blood pressure data that is needed for the clinician to decide about continuing or stopping CPR.

The device according to the present invention solves these problems by offering an additional means to assess the adequacy of the spontaneous pulse in the PPG signal and hence to assess an indicator of cardiogenic output corresponding to ROSC in a reliable manner. In this invention, a device is described that combines a PPG measurement with a blood pressure measurement.

First, one or more PPG signals are obtained by the PPG input. The PPG input is understood as a data interface capable of communicating the PPG signals, i.e. PPG data. The PPG input may be an analogue or digital interface supporting a wired or wireless connection. The PPG signals may be obtained at one or more wavelengths. For example, the incoming light may be ambient light such as sunlight. The PPG signal (i.e. PPG data) may also be obtained using a pulse oximeter monitoring the perfusion of blood to the dermis and/or other tissue of the patient. A typical pulse oximeter comprises a red LED and an infrared LED as light sources and a photodiode for detecting light that has been transmitted through patient tissue, wherein the LEDs and photodetector diode are usually configured to be arranged directly on the skin of the patient, typically on a digit (finger or toe) or earlobe. Other places on the patient may also be suitable. The LEDs may emit light at different wavelengths, the light being diffused through the vascular bed of the patient's skin and received by the photodetector diode. Other simpler versions of a system for obtaining PPG data may be used as well, including those with a single light source of one or more wavelengths.

The gained one or more PPG signals are then analyzed for one or more features indicative of a spontaneous cardiac pulse in the processing unit. In particular, one or more algorithms comprised in the processing unit are configured to detect a spontaneous cardiac pulse during CPR, either during a few-second pause in compressions or during ongoing compressions, wherein a PPG measurement during pauses is more reliable because of less motion artifacts (particularly from chest compressions). Detection of a spontaneous pulse is coupled to a predefined condition with respect to one or more properties of the one or more PPG signals, wherein said properties can be any one of the patient's pulse presence, pulse rate, duration of a predefined pulse rate level, in particular a predefined minimum pulse rate level, variation of inter-pulse intervals over time, his or her pulse strength, the duration of a (stable) pulse detection, and a number of cardiac-induced harmonics detected in the PPG signal.

Algorithms used to analyze PPG signal content to support detection of ROSC may be found in US 2016/0157739 A1 or WO 2017/072055 A1.

When return of a spontaneous pulse is detected in the PPG signal, which is measured (continuously) during CPR, a BP measurement is triggered. Also with respect to the BP measurements performed after detection of a spontaneous pulse there may be different types of algorithms used in the processing unit. The type of algorithms used may particularly depend on the type of PPG sensors and BP sensors employed for the measurements and the timing of the measurements. The BP control signal used to control a BP sensor to measure the patient's blood pressure is generated in the processing unit according to one of said algorithms and forwarded to a BP sensor by the BP output. The BP output is understood as a data interface capable of communicating the BP control signal. It may be an analogue or digital interface supporting a wired or wireless connection. The BP measurement may comprise one or more of mean arterial blood pressure, systolic blood pressure, diastolic blood pressure and digital arterial blood pressure. The BP control signals may cause the BP sensor to measure the blood pressure in the form of a continuous blood-pressure time-trace or in intervals. The obtained BP measurement may then provide the clinician with quantitative information about the status of the circulatory system. In fact, it may serve as an indicator of cardiogenic output and in the following help clinical personnel to assess return of spontaneous circulation, ROSC. The return of ROSC is understood as is known in the art and refers to clinical significance of return of pulse, i.e. the point at which the heart starts to spontaneously generate life-sustaining blood flows.

In general, combining a PPG measurement with a BP system has the following advantage: The PPG measurement provides (continuous) screening whether there is a spontaneous pulse or not. The moment a spontaneous pulse has been detected, a BP measurement can be performed to provide quantitative information about the circulatory system. This can better support the clinician in decision making than PPG only, because it allows the clinician to determine whether the blood pressure is sufficient to stop CPR or not. Apart from that, there is no need for a continuous BP measurement since for the detection of a cardiogenic output, and ROSC in the following, prior to applying a BP measurement it needs to be known whether the heart is beating or not. This can be determined via PPG. Moreover, PPG may support the clinician in decision making further, because besides information about the heart rate, a PPG waveform may also comprise information attributable to further physiological phenomena such as the respiration. And by evaluating the transmittance and/or reflectivity at different wavelengths (typically red and infrared), the blood oxygen saturation (SpO2) can be determined. Hence, this information may also be considered by the processing unit to assess ROSC.

In particular, based on the BP measurement result, a clinician can decide when the blood pressure is sufficiently high so chest compressions can be stopped to further assess potential ROSC, and when the blood pressure is low so chest compressions need to be continued on a beating heart to provide additional support to the heart. Assessing whether CPR actions may be stopped according to a BP measurement may be linked to a predefined condition on the patient's blood pressure. Said condition may, apart from the blood pressure value, comprise a threshold on the duration the patient has at a particular blood pressure. For example, the processing unit may only assess the indicator of cardiogenic output if the diastolic blood pressure (DBP) is measured to be at or above 40 mmHg for at least 30 seconds.

Furthermore, assessing cardiogenic output may also be based on a combination of the BP measurement with the PPG measurement. That is, an indicator of cardiogenic output may be provided by the BP signal properties alone or in combination with PPG signal properties, such as pulse rate. In fact, an indicator of cardiogenic output may be provided by other appropriate vital signs such as a PPG signal property alone, or the combination of an ECG signal property and a PPG signal property. The status of said indicator represents a measure for the probability of ROSC.

In an embodiment of the device, the predefined condition includes one or more of a threshold, a range, an item in a look-up table and a position in a chart.

For example, detection of a spontaneous pulse may only be provided if the patient's pulse has been detected at a pulse strength of at least 0.1% of the patient's average PPG signal level (in the moment of measurement) for at least 30 seconds at a rate of at least 30 beats per minute. That is, according to the present invention the predefined condition defines thresholds for pulse strength, pulse rate, and pulse duration. Additionally, the predefined condition can comprise PPG signal characteristics, such as the number of cardiac-induced harmonics detected in the PPG signal. Likewise, the processing unit may compare the detected pulse rate data, for example, with data in a look-up table of the device's memory. If the data measured comply with the data of said table, the processing unit may decide that a spontaneous pulse has been detected and triggers a BP measurement. The processing unit may also track the PPG signal over time and compare said signal to a PPG signal waveform stored in the memory. In that case the predefined condition may be represented by a match of both signals to a predefined extent. Furthermore, the processing unit may calculate the probability of ROSC based on the measured and/or derived PPG signal parameters.

In another embodiment, the device further comprises an ECG input configured to obtain one or more ECG, electrocardiography, signals, wherein the processing unit is configured to analyze the one or more predefined PPG signal properties using the one or more ECG signals.

The ECG input is understood as a data interface capable of communicating the one or more ECG signals, i.e. ECG data, respectively. The ECG input may be an analogue or digital interface supporting a wired or wireless connection. An (organized) ECG signal may facilitate finding pulses in a PPG signal. When the ECG signal has an organized appearance, QRS-complexes can be distinguished in the signal. This indicates that the heart's electrical activity has been restored again, which is called return of rhythm (ROR). ROR however does not provide information about the heart's mechanical activity. When observing ROR, the heart muscle may still be unable to contract, which occurs in a large group of patients who undergo CPR in the hospital. Therefore, an additional measurement reflecting the heart's mechanical activity is still required. PPG is an easy-to-use, non-invasive method that can show whether or not the heart has resumed beating again. The QRS complexes in the ECG signal as analyzed by the processing unit can facilitate detecting the cardiac-induced pulses in the PPG signal, as the cardiac-induced PPG pulses should follow these QRS complexes when the heart is beating.

Advantageously, the device further comprises a chest compression signal input configured to obtain a chest compression signal indicating one or more of trans-thoracic impedance, chest compression rate, length of inter-compression intervals, chest compression force, chest compression depth, chest compression acceleration and chest compression velocity, wherein the processing unit is configured to analyze the chest compression signal and to recognize and handle or remove artifacts of said chest compression signal from the one or more PPG and / or ECG signals.

While the processing unit may recognize and remove chest compression artifacts from the PPG and / or ECG signals, it may handle said chest compressions otherwise as well. For example, in case the chest compressions influence the PPG and / or ECG signal only up to a predefined level, there may be no need to remove the artifacts of the chest compression signal from the PPG and / or ECG signal. On the other hand, if the chest compression's influence on the one or more PPG signals and / or ECG signal turns out to be higher than said level, the compression artifacts may be reduced or removed from the PPG and / or ECG signal by removing all frequency components related to the measured compression rate and its harmonics, for example. This way, motion artifacts due to chest compressions complicating detection of a cardiac pulse in the PPG signal and QRS complexes in the ECG signal are eliminated or at least reduced. Furthermore, rather than explicitly removing the compressions components from the one or more PPG signals and / or the ECG signal, mere identification of compression-related frequency components can also be sufficient. For instance, when applying spectral analysis to the PPG and / or ECG signals, it can be sufficient to know which frequency components in the spectra are due to chest compressions, so they can be ignored in further analysis of the signal spectrum.

In a further embodiment of the device, the processing unit is configured to analyze the chest compression signal to discriminate a pause in compressions from ongoing compressions and to generate the BP control signal during the pause in compressions or during ongoing compressions.

When BP measurements are triggered to be performed in compression pauses, the PPG measurement can prevent unnecessary interruptions in compressions to perform futile BP measurements. On the other hand, when BP measurements are triggered to be performed during ongoing compressions, a PPG measurement can determine when there is a spontaneous pulse and a BP measurement thus may be started right away to prevent compressions on a beating heart which may cause re-arrest of the heart.

In another embodiment, the device for supporting detection of return of spontaneous circulation further comprises a user input configured to obtain user information from a first user interface, wherein the processing unit is configured to generate the BP control signal and/or to adjust the predefined condition based on the user information, and/or the device further comprises a user output configured to provide ROSC information to a second user interface. Specifically, when the processing unit detects presence of a spontaneous pulse in the PPG signal, user input can be required first to confirm initiating the BP measurement to determine the most appropriate moment to initiate the BP measurement.

User information may comprise BP data of the patient and/or other data of the patient relevant for evaluating the status of an indicator of cardiogenic output and hence for assessing ROSC. User information may particularly be used to adjust thresholds or ranges used in the assessment of the status of the indicator of cardiogenic output, related to, e.g., BP, duration of BP level, PR, duration of a (minimum) PR level, pulse intervals, variations in pulse intervals, pulse presence, duration of pulse presence, pulse strength, the number of cardiac-induced harmonics detected in the PPG signal. These data may also comprise end-tidal-CO2 data, (central-venous) -oxygen-saturation data and/or NIRS data. User information may likewise be information about the CPR protocol used and hence also information comprising the rate of chest compressions applied to the patient. User information may particularly comprise algorithms used in the processing unit to analyze the one or more PPG signals and/or a BP measurement.

ROSC information comprises any one of the following: information about the one or more PPG signals, in particular after removal or reduction of artifacts achieved by using the compression signal, the one or more predefined PPG signal properties, the pulse presence, the pulse rate, the time-duration at a specific (minimum) PR level, the variation of inter-pulse intervals over time, the pulse strength, the duration of (stable) pulse presence, the number of cardiac-induced harmonics detected in the PPG signal, the BP, the rate of chest compressions, chest compression fraction, oxygen saturation and any indicator of cardiogenic output and its status as well as a probability estimate (estimated by the processing unit) of ROSC.

In an advantageous embodiment of the system for supporting detection of return of spontaneous circulation, the BP sensor is comprised of a BP cuff which can be any one of a finger cuff, wrist cuff and arm cuff, and the one or more PPG sensors are configured to be arranged in one of said cuffs, one or more other finger, wrist or arm cuffs or at the patient's finger, earlobe, concha or pinna of the ear, forehead, alar wing or septum of the nose and/or the inside of the mouth, such as the cheek or the tongue.

In particular, the BP cuff may be an inflatable cuff with the external pressure, i.e. the pressure applied by the cuff to the patient's body, being dependent on the level of the cuff inflation. The pressure provided by the cuff is then measured by the BP sensor included in the cuff. The measured external pressure is used for determining a blood pressure, for example the mean arterial blood pressure, the systolic blood pressure, the diastolic blood pressure and/or the digital arterial blood pressure.

In another embodiment, the processing unit is configured to control the cuff pressure continuously or in intervals during ongoing compressions. In particular, the mean cuff pressure may be controlled. In case of a cNIBP measurement, however, the processing unit is used to control the cuff pressure in order to counteract the pressure in vessels in order to keep the blood volume constant (volume-clamping).

In yet another embodiment of the system, at least one of the one or more PPG sensors is configured to be arranged at a skin facing side of the BP cuff, and the processing unit is configured to control a mean cuff pressure applied to the patient by said cuff to maximize an amplitude of the one or more PPG signals of the at least one PPG sensor and/or an oscillation of pressure inside said cuff.

For example, when the PPG measurement is performed distal to a cuff, an algorithm may be chosen which induces a cuff-pressure scan determining at which cuff pressure the spontaneous pulse disappears in the PPG signal. The resulting pressure is a measure for the systolic blood pressure at the site of the cuff.

In a preferred embodiment, the system further comprises one or more ECG sensors configured to measure one or more ECG signals, wherein the processing unit is configured to analyze the one or more ECG signals to facilitate obtaining any one of the one or more PPG signal properties. Alternatively or additionally, the system further comprises a chest compression sensor configured to measure a chest compression signal indicating one or more of trans-thoracic impedance, chest compression rate, length of inter-compression intervals, chest compression force, chest compression depth, chest compression acceleration and chest compression velocity, wherein the processing unit is configured to analyze said chest compression signal and to recognize and handle or remove artifacts of said chest compression signal from the one or more PPG signals and/or ECG signals.

The chest compression sensor may be an accelerometer, a force sensor, a radar, a communication module to an automated compression device or defibrillation pads, with the compression rate being inferred from an impedance signal measured between these pads. In fact, the compression sensor may be any sensor providing a reference on chest compression rate, depth, velocity or acceleration.

The one or more ECG sensors may be any one of a photoelectric sensor, ECG electrodes or a piezoelectric sensor. ECG sensors may be wearable sensors or comprise remote ECG monitoring. The sensors may provide the ECG signals for wired or a wireless communication.

According to another embodiment, the system further comprises a first user interface configured to obtain the user information and/or a second user interface to provide the ROSC information in a visible and/or audible manner.

The first and second user interface may be comprised in separate units or be united in a single unit, i.e. as a single user interface configured to obtain user information and to provide ROSC information. The first and second user interface may comprise one or more displays, buttons, touchscreens, keyboards or other human machine interface means. Hence, user information such as information about invasive-blood-pressure data, (central-venous) oxygen-saturation data, TTI data and/or NIRS data of the patient may be provided via a keyboard input, for example, or directly from another device. The second user interface may also be used to provide a ROSC probability signal based on the probability of ROSC as determined by the processing unit. For example, there may be a bar displayed on a display with the lower end indicating "cardiac arrest" and the upper end indicating "potential ROSC", wherein an arrow aiming at the bar indicates the probability of ROSC, i.e. the status of an indicator of cardiogenic output. The second user interface may likewise prompt an audible signal once a cardiogenic output indicating ROSC has been detected by the processing unit with a predefined probability. Said information may be provided to the user as a real-time feedback.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of a first embodiment of a device for supporting detection of return of spontaneous circulation during cardiopulmonary resuscitation according to the present invention,
Fig. 2 shows a schematic diagram of a second embodiment of a device according to the present invention,
Fig. 3 shows a schematic diagram of a third embodiment of a device according to the present invention,
Fig. 4 shows a schematic diagram of a first embodiment of a system for supporting detection of return of spontaneous circulation during cardiopulmonary resuscitation according to the present invention,
Fig. 5 shows a schematic diagram of a second embodiment of a system for supporting detection of return of spontaneous circulation during cardiopulmonary resuscitation according to the present invention,
Fig. 6 shows an exemplary implementation of a system for supporting detection of return of spontaneous circulation of a patient undergoing CPR according to the present invention,
Fig. 7 shows an embodiment of a display unit of a device according to the present invention, and
Figs. 8A and 8B show different embodiments of BP and PPG sensors according to the present invention.

### DETAILED DESCRIPTION OF THE INVENTION

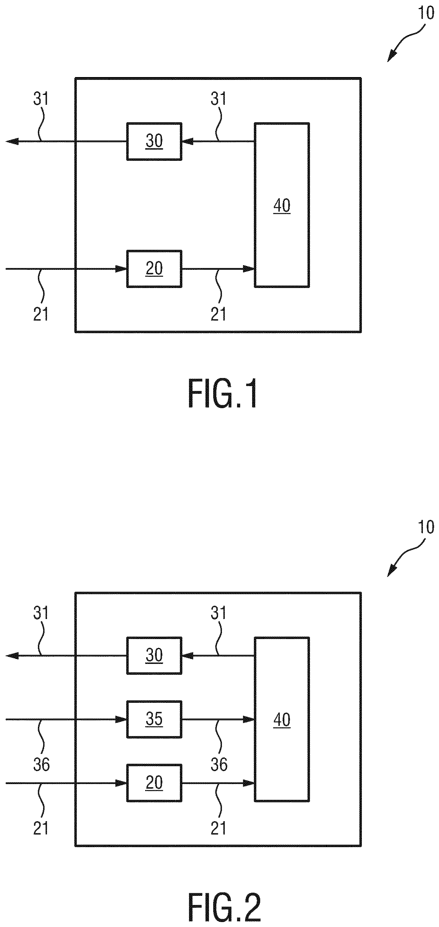

Fig. 1 shows a schematic diagram of a first embodiment of a device 10 for supporting detection of return of spontaneous circulation during cardiopulmonary resuscitation according to the present invention. The device 10 comprises a PPG input 20, a BP output 30 and a processing unit 40. The PPG input 20 is configured to obtain a PPG signal 21 of a patient and to provide said signal 21 to the processing unit 40. Subsequently, the processing unit 40 analyzes the PPG signal 21. In this embodiment, the processing unit 40 is configured to detect a pulse rate of the patient using said signal 21.

Detection of the pulse rate provides information about the rate at which the heart contracts and pumps blood. If the rate is below a predefined threshold, for example 0.5 Hz, the processing unit assumes that there is no ROSC yet. Hence, no other action than continuing the PPG measurement will be performed. Even if the analysis of the PPG signal 21 shows a rate high enough for potential ROSC, chest compressions should be continued if the heart does not yet pump in a stable fashion i.e. if the variation of inter-pulse intervals over time is too high. However, when the heart is pumping again at a stable rate higher than, e.g., 0.5 Hz, it is examined whether there is cardiogenic output by ordering a subsequent blood pressure measurement.

In fact, presence of a stable pulse rate which is sufficiently high is a necessary prerequisite of ROSC: without such a rhythm, there will be no ROSC, and it will be of no use to do a further assessment of cardiogenic output and correspondingly ROSC. On the other hand, presence of a stable, sufficiently high pulse rate in the PPG signal does not directly indicate that there is ROSC, because it does not provide quantitative information about the underlying blood pressure and/or level of perfusion. In other words, return of spontaneous circulation is only given once a perfusing rhythm has been re-established, i.e. when the heart contracts again at a stable rate resulting in a life-sustaining cardiac output. Hence, if the pulse rate is above the predefined threshold and fulfills a stability condition the processing unit 40 generates a BP control signal 31. The BP output 30 then provides the BP control signal for controlling a BP sensor to start measuring the blood pressure of the patient in order to support assessing ROSC.

Fig. 2 shows a schematic diagram of a second embodiment of a device 10 for supporting detection of return of spontaneous circulation during cardiopulmonary resuscitation according to the present invention. Apart from a PPG input 20, a BP output 30 and a processing unit 40, the device 10 also comprises a BP input 35 configured to obtain a BP measurement 36. By analysing the blood pressure signal 36 of the patient the processing unit 40 may assess return of cardiogenic output or a probability of return of spontaneous circulation. ROSC is probably given if, for example, the systolic blood pressure is higher than 60 mmHg. Therefore, the processing unit 40 is configured to check whether the blood pressure is above said or another predefined threshold. If this is the case, the processing unit 40 assesses return of cardiogenic output. A clinician may then stop delivering chest compressions to a patient in order to prevent re-arrest or other damages due to chest compressions and/or to assess ROSC.

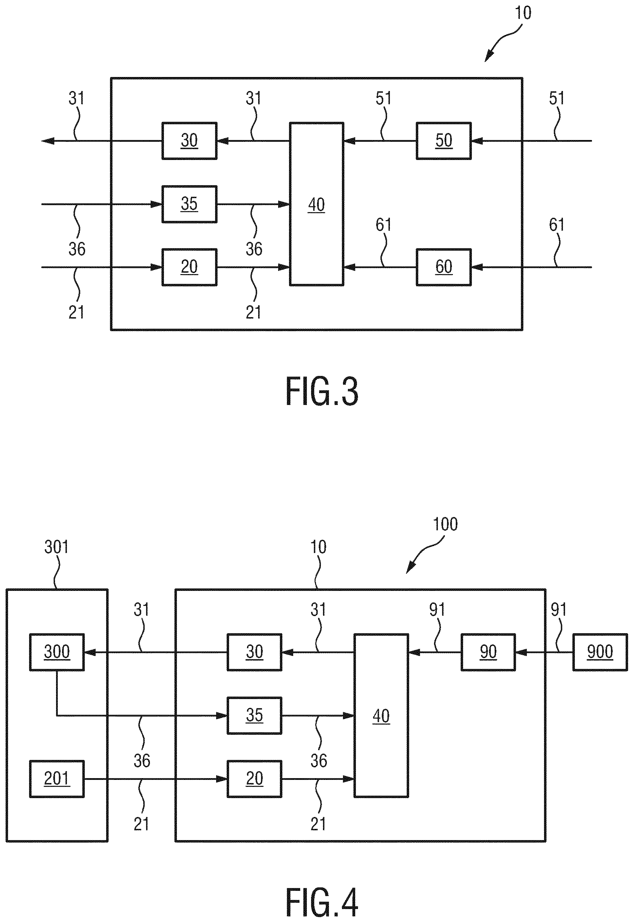

Fig. 3 shows a schematic diagram of a third embodiment of a device 10 according to the present invention. The device 10 comprises a PPG input 20, a BP output 30, a BP input 35, a chest compression signal input 50, a user input 60 and a processing unit 40.

A PPG signal 21 obtained by the PPG input 20 is communicated to the processing unit 40 for analysis. The PPG signal 21 is analyzed for presence of a spontaneous pulse during compressions. Presence of a spontaneous pulse in the PPG signal 21 during compressions can be recognized by an increased complexity in the shape of the PPG signal 21, for example. Alternatively, pulse presence can be detected via spectral analysis of the PPG signal, when two harmonic series can be identified, one of which corresponding to chest compressions and the second of which corresponding to cardiac activity.

Analysis during ongoing compressions also involves using a chest compression reference 51 to detect presence of compressions and to identify the compression rate. The compression reference 51 may be an impedance signal obtained between defibrillation pads attached to the patient, for example. The chest compression signal input 50 is configured to obtain the chest compression signal 51. The chest compression signal 51 indicates the rate of chest compressions delivered to the patient, the variation of inter-compression intervals over time and the compression strength and/or depth, respectively. The processing unit 40 analyzes said properties of the chest compression signal 51. Using the analysis results the processing unit 40 then removes artifacts of the chest compression signal 51 from the PPG signal 21 by removing all frequency components related to the measured compression rate. This way, motion artifacts due to chest compressions complicating detection of a cardiac pulse in the PPG signal 21 are eliminated or attenuated. Alternatively, the processing unit 40 uses the compression rate derived from the compression reference 51, to identify and ignore all spectral components in the PPG signal related to the compressions in order to determine whether the PPG signal contains remaining spectral components related to cardiac activity. E.g., when the processing unit 40 detects two harmonics series in the PPG signal 21, the compression rate information can be used to identify which of these two harmonic series corresponds to chest compressions and which of these two harmonic series corresponds to spontaneous cardiac activity.

The processing unit 40 of this embodiment is further configured to analyze the chest compression signal 51 to discriminate a pause in compressions from ongoing compressions. If the compression signal is determined to indicate a compression rate below a predefined threshold or is determined not to contain compression-related characteristics, the processing unit 40 assesses that there are no chest compressions delivered to the patient, i.e. that there is a pause in compressions. Identifying a pause in compressions based on the compression signal 51 can be used to obtain an alternative detection of presence or absence of the spontaneous cardiac component in the PPG signal 21. Given that there is a pause in compressions and that pulse presence is detected in the PPG signal 21, where pulse presence is detected during ongoing compressions and/or during a pause in compressions, the processing unit 40 generates a BP control signal 31 during the pause in compressions. The BP output 30 takes said signal to control a BP sensor for measuring the blood pressure of the patient. Apart from that, the processing unit 40 may be used to extract information about the quality of CPR components such as compression depth or chest compression fraction.

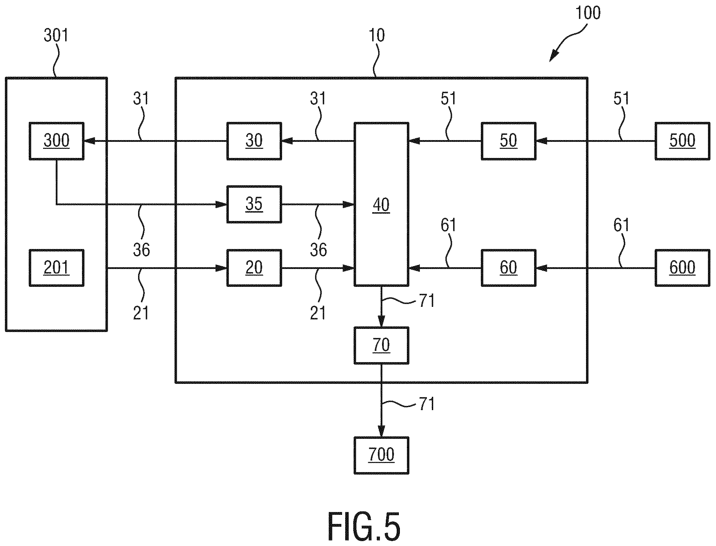

The device 10 for detecting return of spontaneous circulation further comprises a user input 60 configured to obtain user information 61 from a first user interface 600 and to provide ROSC information 71 to a second user interface 700 (see Fig. 5). The processing unit 40 uses the user information 61 in order to generate the BP control signal 31. User information 61 may comprise a confirmation to start the BP measurement, for example. Hence, if the PPG signal analysis results in a high probability for ROSC, the processing unit 40 can first prompt to the user via user interface 700 that cardiogenic output has been detected and request whether a BP measurement should be performed. Only after the user confirmed that performing a BP measurement is appropriate via user interface 600, the BP control signal is generated to initiate the BP measurement. This allows the clinician to determine the moment at which performing a BP measurement is most appropriate. Furthermore, user information provided via user interface 600 can be used to customize thresholds and ranges used in the assessment of the status of the indicator of cardiogenic output. These thresholds and ranges can relate to pulse presence, PR, duration of a (minimum) PR level, pulse intervals, duration of pulse presence, variations in pulse intervals, pulse strength, BP, duration of a specified BP level, the number of cardiac-induced harmonics detected in the PPG signal.

Fig. 4 shows a schematic diagram of a first embodiment of a system 100 for supporting detection of return of spontaneous circulation during cardiopulmonary resuscitation according to the present invention. The system 100 comprises the second embodiment of the device 10 for supporting detection of return of spontaneous circulation, a PPG sensor 201, a BP sensor 300 and an ECG sensor 900.

The BP sensor 300 is integrated in a cuff 301 in this embodiment. Said cuff may be applied to the patient's finger and measure his or her blood pressure during compressions, e.g., by tracking the Mean Arterial Pressure, MAP. Tracking of blood pressure would be performed after presence of pulse was detected in the PPG signal. The PPG measurement module 201 is integrated in the same cuff 301, in particular underneath, i.e. at the skin facing side of, said cuff 301. The PPG and cuff-based pressure measurement can be performed on the middle or proximal phalanx of a finger, in particular on the proximal phalanx of the thumb. The PPG sensor 201 is continuously measuring the PPG signal 21 of the patient, which is measured at, e.g. green, red and / or infrared wavelengths, respectively. The measured PPG signal 21 is communicated to the processing unit 40 for subsequent analysis. In particular, the PPG signal 21 is analyzed for presence of a spontaneous pulse during compressions. Said analysis is supported by the ECG signal 91 provided by the ECG sensor 900 via the ECG input 90. In particular, the ECG signal should first have an organized appearance before analyzing the PPG signal for presence of pulse. That is, the ECG signal should first contain QRS-complexes, which indicates that the heart's electrical activity has been restored again, which is called return of rhythm, ROR. After ROR has occurred, the heart's mechanical activity can be checked for by searching for cardiac-induced pulses in the PPG signal. The QRS complexes in the ECG signal can facilitate detecting the cardiac-induced pulses in the PPG signal, as the cardiac-induced PPG pulses should follow these QRS complexes when the heart is beating. Alternatively, when applying spectral analysis the heart rate (HR) derived from the ECG signal can be used to identify the pulse rate (PR) of the spontaneous pulses in the PPG signal.

After presence of a spontaneous pulse has been detected in the signal 21 with the help of the ECG signal analysis, the cuff-based BP measurement is triggered. To this end, the processing unit 40 is configured to scan the cuff pressure to find the mean arterial pressure (MAP). The MAP is approximated by the cuff pressure at which the spontaneous pulse component in the PPG signal has maximum pulsatility, i.e. maximum amplitude or maximum average signal level. At maximum pulsatility, the vascular compliance is maximum meaning that the vascular wall is unloaded. Unloading of the vascular wall is achieved when the cuff pressure equals the mean blood pressure in the vessel. Alternatively, the pressure oscillations inside the cuff 301 are measured. Then, the MAP is approximated by the cuff pressure at which the pressure oscillations in the cuff 301 have maximum amplitude as measured by the BP sensor 300.

The MAP can also be estimated during cardiac arrest. Then the pulsatility of the compression-induced component in the PPG signal 21 or the amplitude of the compression-induced component in the cuff pressure is maximized.

Alternatively, in another embodiment, the PPG measurement and the BP measurement are not integrated in the same cuff. Here, the PPG measurement could for instance be obtained using a PPG sensor 201 applied to a finger, the forehead, the nasal septum, the alar wing, the earlobe, the concha of the ear, or the pinna of the ear. The BP measurement could be obtained using a cuff 301 applied to the arm, wrist or finger. Once presence of a spontaneous pulse is detected in the PPG signal 21, the cuff-based BP measurement can be triggered. If the cuff 301 would be applied to the upper arm and the PPG measurement would be applied to a digit on the other arm, the BP measurement could run while at the same time the PPG measurement is still being used to track pulse presence. Alternatively, if the PPG measurement would be applied distal to the cuff 301 on the same arm, the PPG measurement could be used to determine BP levels. For instance, when scanning pressure in the arm cuff 301, an estimate of the systolic blood pressure (SBP) would be reached when the spontaneous pulse disappears in the PPG measurement performed distally to the cuff.

Fig. 5 shows a schematic diagram of a second embodiment of a system for supporting detection of return of spontaneous circulation according to the present invention. The system comprises the third embodiment of the device 10, a PPG sensor 201, a BP sensor 300, a chest compression sensor 500, a first user interface 600 and a second user interface 700.

In this embodiment of the system 100, the PPG sensor 201 is incorporated in a finger cuff 301. At the same time, said cuff contains the BP sensor 300. The BP measurement performed by the cuff is a continuous non-invasive blood pressure measurement (cNIBP). A cNIBP measurement needs to be calibrated to find the mean digital arterial pressure around which the processing unit 40 can track variations in blood pressure. In this embodiment, calibration for the cNIPB measurement is performed during ongoing compressions. The mean pressure corresponds to the cuff pressure at which the pulsatility of the PPG signal 21 is maximum (pulsatility = amplitude divided by average signal level). Said pressure is determined by the processing unit 40. At maximum pulsatility, the vascular compliance is maximum which means that the vascular wall is unloaded. Unloading of the vascular wall is achieved when the cuff pressure equals the mean blood pressure in the vessel. Tracking the mean blood pressure and thus the operating point of the cNIBP measurement allows for a more rapid cNIBP measurement in a subsequent pause in compressions, because the correct mean pressure has already been estimated and the cNIBP measurement can start right away. In this embodiment, the operating point of the cNIBP measurement can be tracked continuously over time or at regular intervals in time.

The pulsatility of the PPG signal 21 can be maximized either during cardiac arrest or after detection of a spontaneous pulse in the PPG signal 21. During cardiac arrest, the pulsatility of the compression-induced signal is maximized. After detection of a spontaneous pulse, the pulsatility of the spontaneous pulse component in the PPG signal 21 is maximized. In order to maximize the pulsatility of the spontaneous pulse component, said component is extracted from the PPG signal 21 by removing chest compression artifacts. Removal of chest compression components involves use of a compression reference signal 51 to determine the chest compression rate.

This way, when the PPG-measurement is performed underneath the finger cuff, the mean digital arterial pressure can be tracked during compressions. Tracking the mean digital artery blood pressure during compressions has the following advantages: First, it makes sure that the cNIBP measurement is always at its operating point and ready for use, and second, it improves the quality of the PPG measurement by maximizing the signal amplitude. In fact, when the PPG-measurement is performed underneath the finger-cuff, the cuff can continuously apply a low pressure to assure good signal quality by maximizing the PPG signal pulsatility. This improves the signal-to-noise ratio and quality of the PPG measurement 21. Furthermore, the cuff pressure assures good contact between the PPG sensor 201 and the skin, which reduces motion of the PPG sensor 201 relative to the skin and thereby motion artifacts in the PPG signal 21. Moreover, during cardiac arrest, by tracking the mean digital artery blood pressure the processing unit 40 can assess the effectiveness of the chest compressions.

The second user interface 700 may display the PPG signal time-trace, in particular after removal of chest compression artifacts, the blood pressure measurements (SBP, MAP, DBP), and the cNIBP time-trace, as well as derived information such as an indicator of cardiogenic output to provide clinical decision support. The interface 700 may also show the spontaneous pulse rate derived from the PPG measurement and the oxygen saturation (SpO2) determined from the spontaneous pulse component in the PPG measurements at, e.g., a red and an infrared wavelengths. Furthermore, the user interface 700 may provide audible feedback on the effectiveness of the chest compressions and give advice to the user to improve CPR.

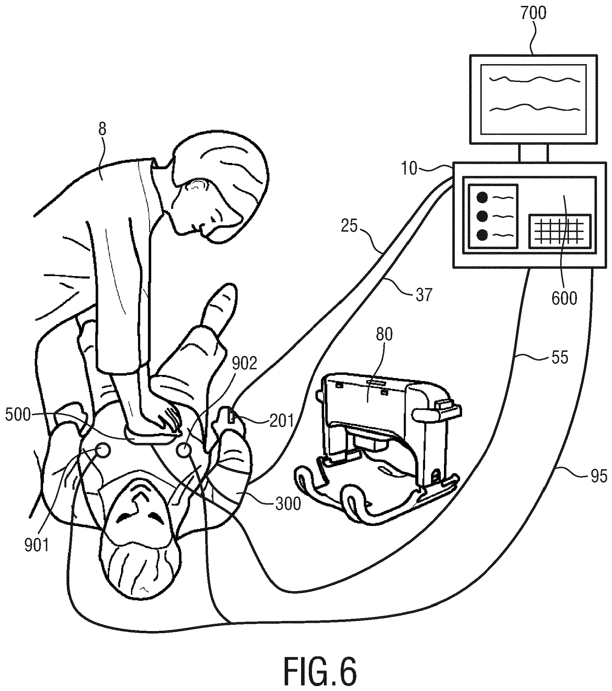

Fig. 6 shows an exemplary implementation of a system 100 for supporting detection of return of spontaneous circulation of a patient undergoing CPR according to the present invention.

The system 100 comprises a device 10, a PPG sensor 201 arranged in a finger cuff, a BP sensor 300 arranged in an inflatable arm cuff, a chest compression sensor 500 attached to the patient's chest and subject to compression, two ECG sensors attached to the patient's chest, a first user interface 600 arranged in the device 10 and a second user interface 700. Chest compressions can be delivered either manually by a clinician 8, for example, or with an automated mechanical device 80.

In this embodiment of the system 100, PPG signal 21 can be measured during a pause in compressions and is used to schedule a BP measurement in the same pause in compressions, wherein the pause in chest compressions can be detected via the compression signal 51 being delivered via a chest compression cable 55 from the chest compression sensor 500 to the device 10. The PPG signal 21 being delivered via a PPG cable 25 from the finger cuff 201 to the device 10 is analyzed for presence of a spontaneous pulse. In order to support said analysis there are used the ECG signals measured by the ECG sensors 901 and 902 and provided to the device 10 via an ECG cable 95. The ECG signals 91 and 92 can be analyzed to detect QRS complexes, which indicate return of rhythm, ROR. After detection of ROR, the PPG signal 21 can be analyzed to find presence of pulse. Here, the ECG signals 91 and 92 can be helpful too, as the cardiac-induced pulses in the PPG signal 21 follow the QRS-complexes in the ECG signals 91 and 92. Alternatively, when applying spectral analysis, the heart rate (HR) derived from the ECG signals 91 and 92 can facilitate determining the pulse rate (PR) in the PPG signal 21. If a spontaneous pulse is detected in the PPG signal 21 during a pause, the cuff-based BP measurement is scheduled. The BP measurement can then be automatically performed directly after the detection of a spontaneous pulse.

Alternatively, the system can prompt to the clinician via a display or with an audible signal of the second user interface 700 that a spontaneous pulse has been detected and suggest to start the cuff-based BP measurement directly after pressing a button of the first user interface 600. In the latter case, the clinician 8 has the option to postpone the BP measurement, in case it is already clear to the clinician that the pulse cannot yet be life-sustaining. Furthermore, the system 100 may also measure and analyze PPG signal 21 for pulse presence during ongoing compressions. In that case, a BP measurement can be performed during the next pause in compressions, either automatically or after a confirmation from the user via user interface 600. If compressions are delivered in cycles of two minutes, for instance, as is customary in CPR, the BP measurement can be scheduled for the short pause directly following the two minute cycle of compressions. Alternatively, the system can prompt to the clinician 8 that a spontaneous pulse has been detected and suggest starting the BP measurement directly after pressing a button. In the latter case the clinician 8 has the option not to wait for the next pause in the protocol, but deviate from the standard protocol to personalize the treatment to a patient.

The BP measurement can be performed to measure systolic, diastolic and/or mean arterial blood pressure. The type of blood pressure to be measured may be decided by the user 8 using the user interface 600. In particular, the user interface 600 may be configured to enable the user to change the inflation and/or deflation rate of a BP cuff. The user may also provide user information with respect to the local hospital CPR protocol he/she uses. The measured BP signal 31 is provided to the device 10 via a BP cable 37. Using the BP measurements it is decided by the device 10 whether a cardiogenic output has been achieved (or how high the probability is for ROSC). The thresholds and ranges used in the assessment of the status of the indicator of cardiogenic output can be customized by the user via user interface 600. These thresholds and ranges can relate to pulse presence, PR, duration of a (minimum) PR level, pulse intervals, duration of pulse presence, variations in pulse intervals, pulse strength, number of cardiac-induced harmonics detected in the PPG signal, BP, duration of a specified BP level. If the status assessment of the indicator of cardiogenic output decides for pulse presence, the processing unit 40 provides corresponding information to the display 700 indicating to the clinician 8 that a further assessment of ROSC is advised. If ROSC has probably not been achieved, however, a new two-minute block of CPR is suggested via the display or is directly initiated to be performed by the mechanical CPR device 80.

The PPG measurement can be performed on the same arm as where the BP measurement is performed distal to the cuff 301, or on the other arm not used for BP measurements. A BP measurement would be performed after pulse presence has been detected in the PPG signal 21. In the first case, where the PPG measurement is performed distal to the cuff 301, the PPG measurement can no longer be used for pulse presence detection during the BP measurement. However, in this case the PPG measurement could be used to determine BP levels. For instance, when scanning pressure in the arm cuff 301, an estimate of the systolic blood pressure (SBP) would be reached when the spontaneous pulse disappears in the PPG measurement performed distally to the cuff 301. Alternatively, when the PPG measurement is performed on a digit on the other arm than where the BP measurement is performed, the BP measurement could run while at the same time the PPG measurement is still being used to track pulse presence.

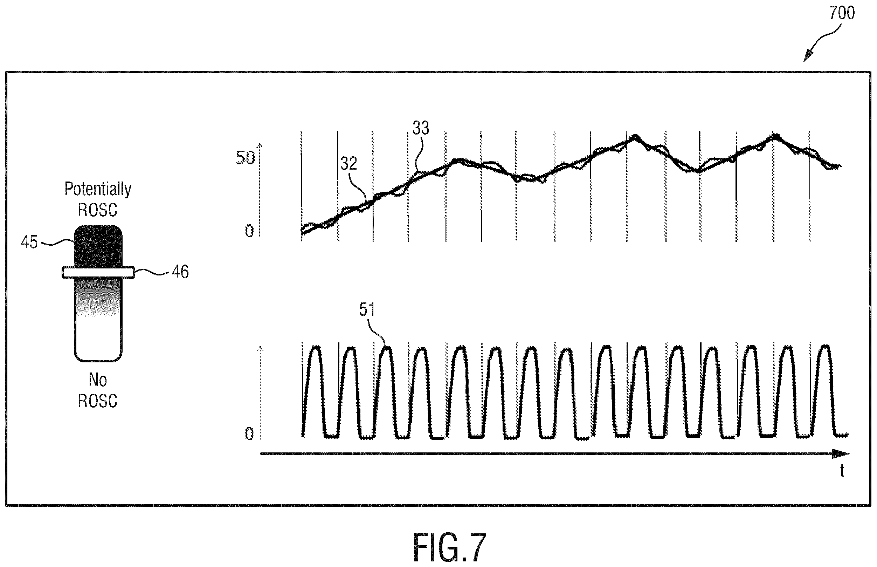

Fig. 7 shows an exemplary implementation of a second user interface 700 of a system 100 according to the present invention.

In this implementation, the second user interface 700 is a display unit showing tracking of a diastolic blood pressure signal 32, oscillations 33 of said signal and a chest compression signal 51 over time. The diastolic blood pressure 32 and the oscillations 33 are depicted on a 0-50 mmHg pressure scale. The oscillations 33 can be seen to be in direct relation with the chest compression signal 51.

The processing unit 40 processes the measured PPG and BP data 21 and 31 to determine the status of an indicator of cardiogenic output, which has a continuous scale ranging from "cardiac arrest" to "potential ROSC". Said scale is also displayed on the display unit 700 as a ROSC probability bar 45. An indicator 46 indicates in real-time the probability of ROSC as assessed by the processing unit. Hence, the user is immediately informed once a potentially life-sustaining pulse has been detected and may therefore decide to stop CPR actions on a restarted heart and further assess the potential ROSC.

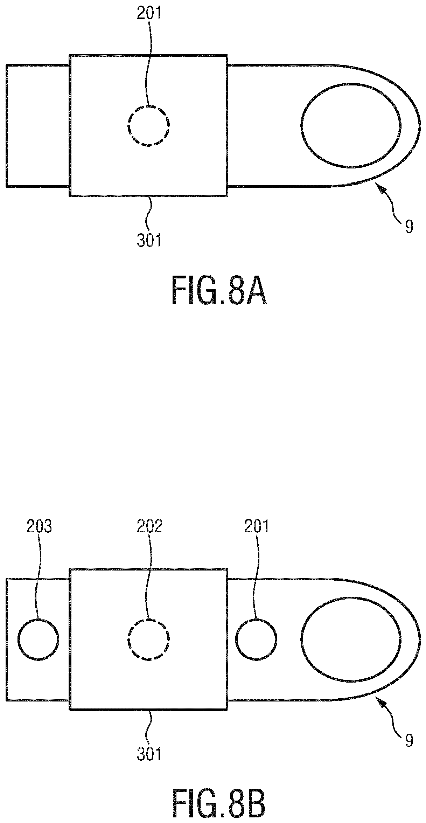

Figs. 8A and 8B show different embodiments of BP and PPG sensors according to the present invention.

Fig. 8A shows a finger cuff 301 wrapped around a finger 9 comprising both a PPG sensor 201 and a BP sensor 300. In this embodiment, a continuous non-invasive blood pressure measurement is achieved via a volume clamping cuff. The PPG sensor 201 integrated in the cuff comprises a red LED and an infrared LED as light sources and a photodiode for detecting light emitted by said sources and reflected by / transmitted through the patient's skin. Here the PPG measurement is used continuously to detect the presence of pulse. Once a pulse has been detected, the blood pressure measurement is triggered. As the BP measurement is based on the volume clamping method, the PPG measurement can no longer be used during the BP measurement. The volume-clamping based method can be used continuously or intermittently after pulse presence has been detected via PPG. In case of intermittent BP measurements, the PPG measurement can be used alternatingly with the BP measurement to keep tracking pulse presence.

For the determination of certain vital signs certain light colour ranges for the PPG signal 21 have shown best results. Furthermore, while the patient's pulse rate can be determined using a single wavelength, corresponding to, e.g., red, infrared or green, clear information about the oxygenation level of the blood (SpO2) providing further indication for ROSC can only be given using at least two different wavelengths for analysis. For example, the relative amplitude ratio of the wavelength interval around 650nm (red) and 840nm or 900nm (infrared) has been shown to give reliable results for SpO2.

In the embodiment of BP and PPG sensors shown in Fig. 8B three PPG sensors 201, 202 and 203 are combined with a finger-cuff 301 for BP measurements, i.e. comprising a BP sensor, wrapped around a patient's finger 9. While the first PPG sensor 201 is arranged on the finger's skin distal to the cuff 301, the second PPG sensor 202 is arranged at a skin-facing side inside the cuff 301, and the third PPG sensor 203 is located on the patient's skin proximal to the cuff 301.

In this embodiment, the spontaneous pulse component in the signal of the first PPG sensor 201 is used to determine the systolic blood pressure (SBP). After a spontaneous pulse has been detected in the first PPG signal 21, the cuff-based BP measurement is triggered. The cuff-pressure is scanned to determine the SBP. This can be done during compressions or during a pause in compressions. If done during compressions, the SBP can be obtained in various ways. The cuff 301 is inflated until the spontaneous pulse component disappears in the first PPG signal 21, where the spontaneous pulse component has been extracted from the first PPG signal 21. The cuff pressure at which this happens is an estimate of the SBP. Alternatively, when the spontaneous pulse component is not explicitly extracted from the PPG signal 21, the SBP is approximated by the cuff-pressure at which the PPG signal amplitude / variance is minimized, as some compression artifacts in the PPG signal may not result from compression-induced pressure-waves propagating through the vessels but rather result from compression-induced sensor-tissue motion. Next, a blood pressure waveform can be obtained, by using the cuff 301 with the second PPG sensor 202 with a volume-clamping method for measuring cNIBP during a pause in compressions. The pulse pressure measured by cNIBP is a good estimate, but the absolute level of the cNIBP waveform needs to be calibrated. The calibration will here be performed using the SBP measurement obtained with the first PPG sensor 201. The systolic pressure in the cNIBP waveform is set equal to the SBP estimate obtained with the first PPG sensor 201. This allows for a measurement of the diastolic blood pressure (DBP) as well. During use of the volume-clamping method in the compression pause, the first PPG sensor 201 may provide unreliable results because the pulse signal is influenced upstream by a cuff. Therefore, during the cNIBP measurement, a third PPG sensor 203 can be used for PR and SpO2 measurements.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A device (10) for supporting detection of return of spontaneous circulation during cardiopulmonary resuscitation, the device comprising
- a PPG input (20) configured to obtain one or more photoplethysmography, PPG, signals (21) of a patient;
- a BP output (30) configured to provide a BP control signal (31) for controlling a BP sensor to start measuring blood pressure, BP, of the patient;
- a BP input (35) configured to obtain one or more BP measurements (36);
and
- a processing unit (40) configured to analyze one or more predefined PPG signal properties of the one or more PPG signals (21, 22, 23) and to generate the BP control signal (31) if one or more of the PPG signal properties fulfill a predefined condition,
wherein the one or more PPG signal properties correspond to at least pulse rate, pulse strength, and duration of the pulse presence of the patient, and wherein the predefined condition comprises respective thresholds for pulse rate, pulse strength, and pulse presence duration, and wherein the predefined condition is for detecting return of a spontaneous pulse;
wherein the processing unit (40) is configured to assess a status of an indicator of cardiogenic output indicating return of spontaneous circulation based on a combination of the one or more PPG signals (21, 22, 23) with the BP measurements.

2. The device (10) according to claim 1,
wherein the one or more PPG signal properties correspond to one or more of duration at a predefined pulse rate level, variation of inter-pulse intervals over time, a number of cardiac-induced harmonics, and a PPG waveform.

3. The device (10) according to any of claims 1-2,
wherein the predefined condition includes one or more of a threshold, a range, an item in a look-up table, a position in a chart, and a comparison with a stored PPG waveform.

4. The device (10) according to any one of the preceding claims,
further comprising an ECG input (90) configured to obtain one or more ECG, electrocardiography, signals (91),
wherein the processing unit (40) is configured to analyze the one or more predefined PPG signal properties using the one or more ECG signals (91).

5. The device (10) according to any one of the preceding claims,
further comprising a chest compression signal input (50) configured to obtain a chest compression signal (51) indicating one or more of trans-thoracic impedance, chest compression rate, length of inter-compression intervals, chest compression force, chest compression depth, chest compression acceleration and chest compression velocity, wherein the processing unit (40) is configured to analyze the chest compression signal (51) and to recognize and handle or remove artifacts of said chest compression signal (51) from the one or more PPG signals (21, 22, 23) and / or the ECG signals (91); and optionally wherein the processing unit (40) is configured to analyze the chest compression signal (51) to discriminate a pause in compressions from ongoing compressions and to generate the BP control signal (31) during the pause in compressions or during ongoing compressions.

6. The device (10) according to any one of the preceding claims,
further comprising a user input (60) configured to obtain user information (61) from a first user interface, wherein the processing unit (40) is configured to generate the BP control signal (31) and/or to adjust the predefined condition based on the user information (61), and/or further comprising a user output (70) configured to provide ROSC information (71) to a second user interface.

7. A system (100) for supporting detection of return of spontaneous circulation during cardiopulmonary resuscitation, the system (100) comprising
- one or more PPG sensors (201, 202, 203) configured to measure one or more PPG signals (21, 22, 23);
- the device (10) as claimed in claim 1 configured to generate a BP control signal (31) by use of the one or more PPG signals (21, 22, 23); and
- a BP sensor (300) configured to perform BP measurements (36) based on the BP control signal (31);
wherein the device (10) is configured to assess a status of an indicator of cardiogenic output indicating return of spontaneous circulation based on a combination of the one or more PPG signals (21, 22, 23) with the BP measurements.

8. The system (100) according to claim 7,
wherein the BP sensor (300) is comprised of a BP cuff (301) which can be any one of a finger cuff, wrist cuff and arm cuff, and
wherein the one or more PPG sensors (201, 202, 203) are configured to be arranged in one of said cuffs, one or more other finger, wrist or arm cuffs or at the patient's finger, earlobe, concha or pinna of the ear, forehead, alar wing or septum of the nose and/or the inside of the mouth, such as the cheek or the tongue.

9. The system (100) according to claim 8,
wherein the processing unit (40) is configured to control the cuff pressure continuously or in intervals during ongoing compressions or during pauses in compressions.

10. The system (100) according to claims 7 to 9,
wherein at least one of the one or more PPG sensors (201, 202, 203) is configured to be arranged at a skin facing side of the BP cuff (301), and
wherein the processing unit (40) is configured to control a mean cuff pressure applied to the patient by said cuff (301) to maximize an amplitude of the one or more PPG signals (21, 22, 23) of the at least one PPG sensor (201, 202, 203) and/or an oscillation of pressure inside said cuff (301).

11. The system (100) according to any one of claims 7 to 10,
further comprising one or more ECG sensors (900, 901, 902) configured to measure one or more ECG signals (91), wherein the processing unit (40) is configured to analyze the one or more ECG signals (91) to facilitate obtaining any one of the one or more PPG signal properties, and/or
further comprising a chest compression sensor (500) configured to measure a chest compression signal (51) indicating one or more of trans-thoracic impedance, chest compression rate, length of inter-compression intervals, chest compression force, chest compression depth, chest compression acceleration and chest compression velocity,
wherein the processing unit (40) is configured to analyze said chest compression signal (51) and to recognize and handle or remove artifacts of said chest compression signal (51) from the one or more PPG signals (21, 22, 23) and/or ECG signals (91).

12. The system (100) according to any one of claims 7 to 11,
further comprising a first user interface (600) configured to obtain the user information (61) and/or a second user interface (700) to provide the ROSC information (71) in a visible and/or audible manner.

13. A method for supporting detection of return of spontaneous circulation during cardiopulmonary resuscitation, the method comprising
- obtaining one or more photoplethysmography, PPG, signals (21, 22, 23) of a patient;
- providing a BP control signal (31) for controlling a BP sensor (300) to start measuring blood pressure, BP, of the patient;
- obtain one or more BP measurements (36);
- analyzing one or more predefined PPG signal properties of the one or more PPG signals (21, 22, 23) and generating the BP control signal (31) if one or more of the PPG signal properties fulfill a predefined condition,
wherein the one or more PPG signal properties correspond to at least pulse rate, pulse strength, and duration of the pulse presence of the patient, and wherein the predefined condition comprises respective thresholds for pulse rate, pulse strength, and pulse presence duration, and wherein the predefined condition is for detecting return of a spontaneous pulse; and
assessing a status of an indicator of cardiogenic output indicating return of spontaneous circulation based on a combination of the one or more PPG signals (21, 22, 23) with the BP measurements.

14. The method of claim 13,
wherein the one or more PPG signal properties correspond to one or more of duration at a predefined pulse rate level, variation of inter-pulse intervals over time, a number of cardiac-induced harmonics and a PPG waveform.

15. A computer program comprising program code means for causing a computer to carry out the steps of the method as claimed in any of claims 13-14 when said computer program is carried out on the computer.

## Patentansprüche

1. Vorrichtung (10) zum Unterstützen einer Erfassung einer Rückkehr eines Spontankreislaufs während einer kardiopulmonalen Wiederbelebung, die Vorrichtung umfassend
- einen PPG-Eingang (20), der konfiguriert ist, um ein oder mehrere Signale von Photoplethysmographie, PPG, (21) eines Patienten zu erlangen;
- einen BP-Ausgang (30), der konfiguriert ist, um ein BP-Steuersignal (31) zum Steuern eines BP-Sensors bereitzustellen, um ein Messen des Blutdrucks, BP, des Patienten zu beginnen;
- einen BP-Eingang (35), der konfiguriert ist, um eine oder mehrere BP-Messungen (36) zu erlangen; und
- eine Verarbeitungseinheit (40), die konfiguriert ist, um eine oder mehrere vordefinierte PPG-Signaleigenschaften des einen oder der mehreren PPG-Signale (21, 22, 23) zu analysieren und das BP-Steuersignal (31) zu erzeugen, wenn eine oder mehrere der PPG-Signaleigenschaften eine vordefinierte Bedingung erfüllen, wobei die eine oder die mehreren PPG-Signaleigenschaften mindestens der Pulsrate, der Pulsstärke und der Dauer der Pulsanwesenheit des Patienten entsprechen, und wobei die vordefinierte Bedingung jeweilige Schwellenwerte für die Pulsrate, die Pulsstärke und die Pulsanwesenheitsdauer umfasst, und wobei die vordefinierte Bedingung zum Erfassen der Rückkehr eines spontanen Pulses ist; wobei die Verarbeitungseinheit (40) konfiguriert ist, um einen Zustand eines Indikators für kardiogenen Ausgang, der eine Rückkehr eines Spontankreislaufs angibt, basierend auf einer Kombination des einen oder der mehreren PPG-Signale (21, 22, 23) mit den BP-Messungen zu beurteilen.

2. Vorrichtung (10) nach Anspruch 1, wobei die eine oder die mehreren PPG-Signaleigenschaften einer oder mehreren von einer Dauer bei einem vordefinierten Pulsfrequenzniveau, Variation der Interpulsintervalle über die Zeit, Anzahl der kardial induzierten Oberwellen und PPG-Wellenform entsprechen.

3. Vorrichtung (10) nach einem der Ansprüche 1-2, wobei die vordefinierte Bedingung eines oder mehrere von einem Schwellenwert, einem Bereich, einem Objekt in einer Nachschlagetabelle, einer Position in einem Diagramm und einem Vergleich mit einer gespeicherten PPG-Wellenform umfasst.

4. Vorrichtung (10) nach einem der vorherigen Ansprüche, ferner umfassend einen EKG-Eingang (90), der konfiguriert ist, um ein oder mehrere EKG-, Elektrokardiographie-, Signale (91) zu erlangen, wobei die Verarbeitungseinheit (40) konfiguriert ist, um die eine oder die mehreren vordefinierten PPG-Signaleigenschaften unter Verwendung des einen oder der mehreren EKG-Signale (91) zu analysieren.

5. Vorrichtung (10) nach einem der vorherigen Ansprüche, ferner umfassend einen Brustkompression-Signaleingang (50), der konfiguriert ist, um ein Brustkompressionssignal (51) zu erlangen, das eines oder mehrere von trans-thorakaler Impedanz, Brustkompressionsrate, Länge der Interkompressionsintervalle, Brustkompressionskraft, Brustkompressionstiefe, Brustkompressionsbeschleunigung und Brustkompressionsgeschwindigkeit angibt, wobei die Verarbeitungseinheit (40) konfiguriert ist, um das Brustkompressionssignal (51) zu analysieren und Artefakte des Brustkompressionssignals (51) aus dem einen oder den mehreren PPG-Signalen (21, 22, 23) und/oder den EKG-Signalen (91) zu erkennen und zu behandeln oder zu entfernen; und optional, wobei die Verarbeitungseinheit (40) konfiguriert ist, um das Brustkompressionssignal (51) zu analysieren, um eine Kompressionspause von laufenden Kompressionen zu unterscheiden und das BP-Steuersignal (31) während der Kompressionspause oder während laufender Kompressionen zu erzeugen.

6. Vorrichtung (10) nach einem der vorherigen Ansprüche, ferner umfassend einen Benutzereingang (60), der konfiguriert ist, um Benutzerinformationen (61) von einer ersten Benutzeroberfläche zu erlangen, wobei die Verarbeitungseinheit (40) konfiguriert ist, um das BP-Steuersignal (31) zu erzeugen und/oder die vordefinierte Bedingung basierend auf den Benutzerinformationen (61) einzustellen, und/oder ferner umfassend einen Benutzerausgang (70), der konfiguriert ist, um ROSC-Informationen (71) an eine zweite Benutzeroberfläche bereitzustellen.

7. System (100) zum Unterstützen einer Erfassung einer Rückkehr eines Spontankreislaufs während einer kardiopulmonalen Wiederbelebung, das System (100) umfassend
- einen oder mehrere PPG-Sensoren (201, 202, 203), die zum Messen eines oder mehrerer PPG-Signale (21, 22, 23) konfiguriert sind;
- die Vorrichtung (10) nach Anspruch 1, die konfiguriert ist, um durch Verwenden des einen oder der mehreren PPG-Signale (21, 22, 23) ein BP-Steuersignal (31) zu erzeugen; und
- einen BP-Sensor (300), der konfiguriert ist, um BP-Messungen (36) basierend auf dem BP-Steuersignal (31) auszuführen; wobei die Vorrichtung (10) konfiguriert ist, um einen Zustand eines Indikators für kardiogenen Ausgang, der eine Rückkehr eines Spontankreislaufs angibt, basierend auf einer Kombination des einen oder der mehreren PPG-Signale (21, 22, 23) mit den BP-Messungen zu beurteilen.

8. System (100) nach Anspruch 7, wobei der Blutdrucksensor (300) aus einer BP-Manschette (301) besteht, die eine Fingermanschette, eine Handgelenkmanschette oder eine Armmanschette sein kann, und wobei der eine oder die mehreren PPG-Sensoren (201, 202, 203) konfiguriert sind, um in einer der Manschetten, einer oder mehreren anderen Finger-, Handgelenk- oder Armmanschetten oder an dem Finger des Patienten, an dem Ohrläppchen, an der Ohrmuschel oder an der Ohrmuschel, an der Stirn, an dem Nasenflügel oder an der Nasenscheidewand und/oder an der Innenseite des Munds, wie der Wange oder der Zunge, angeordnet zu sein.

9. System (100) nach Anspruch 8, wobei die Verarbeitungseinheit (40) konfiguriert ist, um den Manschettendruck während laufender Kompressionen oder während Kompressionspausen kontinuierlich oder in Intervallen zu steuern.

10. System (100) nach einem der Ansprüche 7 bis 9, wobei mindestens einer von dem einen oder den mehreren PPG-Sensoren (201, 202, 203) konfiguriert ist, um an einer der hautzugewandten Seite der BP-Manschette (301) angeordnet zu sein, und wobei die Verarbeitungseinheit (40) konfiguriert ist, um einen mittleren Manschettendruck zu steuern, der durch die Manschette (301) auf den Patienten ausgeübt wird, um eine Amplitude des einen oder der mehreren PPG-Signale (21, 22, 23) des mindestens einen PPG-Sensors (201, 202, 203) und/oder eine Druckschwingung innerhalb der Manschette (301) zu maximieren.

11. System (100) nach einem der Ansprüche 7 bis 10, ferner umfassend einen oder mehrere EKG-Sensoren (900, 901, 902), die konfiguriert sind, um ein oder mehrere EKG-Signale (91) zu messen, wobei die Verarbeitungseinheit (40) konfiguriert ist, um das eine oder die mehreren EKG-Signale (91) zu analysieren, um ein Erlangen eines beliebigen von dem einen oder den mehreren PPG-Signaleigenschaften zu ermöglichen, und/oder ferner umfassend einen Brustkompressionssensor (500), der konfiguriert ist, um ein Brustkompressionssignal (51) zu messen, das eines oder mehrere von trans-thorakaler Impedanz, Brustkompressionsrate, Länge der Interkompressionsintervalle, Brustkompressionskraft, Brustkompressionstiefe, Brustkompressionsbeschleunigung und Brustkompressionsgeschwindigkeit angibt, wobei die Verarbeitungseinheit (40) konfiguriert ist, um das Brustkompressionssignal (51) zu analysieren und Artefakte des Brustkompressionssignals (51) aus dem einen oder den mehreren PPG-Signalen (21, 22, 23) und/oder den EKG-Signalen (91) zu erkennen und zu behandeln oder zu entfernen.

12. System (100) nach einem der Ansprüche 7 bis 11, ferner umfassend eine erste Benutzeroberfläche (600), die konfiguriert ist, um die Benutzerinformationen (61) zu erlangen, und/oder eine zweite Benutzeroberfläche (700), die die ROSC-Informationen (71) auf sichtbare und/oder hörbare Weise bereitstellt.

13. Verfahren zum Unterstützen eines Erfassens einer Rückkehr eines Spontankreislaufs während kardiopulmonaler Wiederbelebung, das Verfahren umfassend
- Erlangen eines oder mehrerer Signale von Photoplethysmographie, PPG, (21, 22, 23) eines Patienten;
- Bereitstellen eines BP-Steuersignals (31) zum Steuern eines BP-Sensors (300), um ein Messen des Blutdrucks, BP, des Patienten zu beginnen;
- Erlangen einer oder mehrerer BP-Messungen (36);
- Analysieren einer oder mehrerer vordefinierter PPG-Signaleigenschaften des einen oder der mehreren PPG-Signale (21, 22, 23) und Erzeugen des BP-Steuersignals (31), wenn eine oder mehrere der PPG-Signaleigenschaften eine vordefinierte Bedingung erfüllen, wobei die eine oder die mehreren PPG-Signaleigenschaften mindestens der Pulsrate, der Pulsstärke und der Dauer der Pulsanwesenheit des Patienten entsprechen, und wobei die vordefinierte Bedingung jeweilige Schwellenwerte für die Pulsrate, die Pulsstärke und die Pulsanwesenheitsdauer umfasst, und wobei die vordefinierte Bedingung zum Erfassen der Rückkehr eines spontanen Pulses ist; und Bewerten eines Zustands eines Indikators für kardiogenen Ausgang, der eine Rückkehr eines Spontankreislaufs angibt, basierend auf einer Kombination des einen oder der mehreren PPG-Signale (21, 22, 23) mit den BP-Messungen.

14. Verfahren nach Anspruch 13, wobei die eine oder mehrere PPG-Signaleigenschaften einer oder mehreren von einer Dauer mit einem vordefinierten Pulsfrequenzniveau, Variation der Interpulsintervalle über die Zeit, Anzahl der kardial induzierten Oberwellen und PPG-Wellenform entsprechen.

15. Computerprogramm, umfassend Programmcode-Einrichtungen, um einen Computer zu veranlassen, die Schritte des Verfahrens nach einem der Ansprüche 13 bis 14 durchzuführen, wenn das Computerprogramm auf dem Computer durchgeführt wird.

## Revendications

1. Dispositif (10) pour supporter la détection de retour de circulation spontanée pendant la réanimation cardiopulmonaire, le dispositif comprenant
- une entrée PPG (20) configurée pour obtenir un ou plusieurs signaux de photopléthysmographie, PPG (21) d'un patient;
- une sortie BP (30) configurée pour fournir un signal de commande BP (31) pour commander un capteur BP pour commencer à mesurer la pression sanguine, BP, du patient;
- une entrée BP (35) configurée pour obtenir une ou plusieurs mesures BP (36); et
- une unité de traitement (40) configurée pour analyser une ou plusieurs propriétés de signal PPG prédéfinies de l'un ou des plusieurs signaux PPG (21, 22, 23) et pour générer le signal de commande BP (31) si une ou plusieurs des propriétés de signal PPG remplissent une condition prédéfinie, dans lequel l'une ou les plusieurs propriétés de signal PPG correspondent à au moins la fréquence du pouls, la force du pouls et la durée de la présence du pouls du patient, et dans lequel la condition prédéfinie comprend des seuils respectifs pour la fréquence du pouls, la force du pouls et la durée de présence du pouls, et dans lequel la condition prédéfinie sert à détecter le retour d'un pouls spontané; dans lequel l'unité de traitement (40) est configurée pour évaluer un état d'un indicateur de sortie cardiogénique indiquant le retour de circulation spontanée sur la base d'une combinaison de l'un ou des plusieurs signaux PPG (21, 22, 23) avec les mesures BP.

2. Dispositif (10) selon la revendication 1, dans lequel l'une ou les plusieurs propriétés de signal PPG correspondent à une ou plusieurs parmi une durée à un niveau de fréquence du pouls prédéfini, une variation d'intervalles inter-pouls dans le temps, un certain nombre d'harmoniques induites par le coeur et une forme d'onde PPG.

3. Dispositif (10) selon l'une quelconque des revendications 1-2, dans lequel la condition prédéfinie inclut un ou plusieurs parmi un seuil, une plage, un élément dans une table de consultation, une position dans un graphique et une comparaison avec une forme d'onde PPG stockée.

4. Dispositif (10) selon l'une quelconque des revendications précédentes, comprenant en outre une entrée ECG (90) configurée pour obtenir un ou plusieurs signaux ECG, électrocardiographie (91), dans lequel l'unité de traitement (40) est configurée pour analyser l'une ou les plusieurs propriétés de signal PPG prédéfinies en utilisant l'un ou les plusieurs signaux ECG (91).

5. Dispositif (10) selon l'une quelconque des revendications précédentes, comprenant en outre une entrée de signal de compression thoracique (50) configurée pour obtenir un signal de compression thoracique (51) indiquant un ou plusieurs parmi l'impédance transthoracique, le taux de compression thoracique, la longueur des intervalles intercompressions, la force de compression thoracique, la profondeur de compression thoracique, l'accélération de compression thoracique et la vitesse de compression thoracique, dans lequel l'unité de traitement (40) est configurée pour analyser le signal de compression thoracique (51) et pour reconnaître et gérer ou supprimer des artéfacts dudit signal de compression thoracique (51) à partir de l'un ou des plusieurs signaux PPG (21, 22, 23) et/ou des signaux ECG (91) ; et facultativement dans lequel l'unité de traitement (40) est configurée pour analyser le signal de compression thoracique (51) pour discriminer une pause dans les compressions des compressions en cours et pour générer le signal de commande BP (31) pendant la pause dans les compressions ou pendant les compressions en cours.

6. Dispositif (10) selon l'une quelconque des revendications précédentes, comprenant en outre une entrée utilisateur (60) configurée pour obtenir des informations utilisateur (61) à partir d'une première interface utilisateur, dans lequel l'unité de traitement (40) est configurée pour générer le signal de commande BP (31) et/ou pour ajuster la condition prédéfinie sur la base des informations utilisateur (61), et/ou comprenant en outre une sortie utilisateur (70) configurée pour fournir des informations ROSC (71) à une seconde interface utilisateur.

7. Système (100) pour supporter la détection de retour de circulation spontanée pendant la réanimation cardiopulmonaire, le système (100) comprenant
- un ou plusieurs capteurs PPG (201, 202, 203) configurés pour mesurer un ou plusieurs signaux PPG (21, 22, 23);
- le dispositif (10) selon la revendication 1 configuré pour générer un signal de commande BP (31) en utilisant l'un ou les plusieurs signaux PPG (21, 22, 23); et
- un capteur BP (300) configuré pour effectuer des mesures BP (36) sur la base du signal de commande BP (31); dans lequel le dispositif (10) est configuré pour évaluer un état d'un indicateur de sortie cardiogénique indiquant le retour de circulation spontanée sur la base d'une combinaison de l'un ou des plusieurs signaux PPG (21, 22, 23) avec les mesures BP.

8. Système (100) selon la revendication 7, dans lequel le capteur BP (300) est composé d'un brassard BP (301) qui peut être l'un quelconque parmi un brassard de doigt, un brassard de poignet et un brassard de bras, et dans lequel l'un ou les plusieurs capteurs PPG (201, 202, 203) sont configurés pour être agencés dans un desdits brassards, un ou plusieurs autres brassards de doigt, de poignet ou de bras ou au niveau du doigt, du lobe de l'oreille, de la conque ou du pavillon de l'oreille du patient, le front, l'aile alaire ou le septum du nez et/ou l'intérieur de la bouche, comme la joue ou la langue.

9. Système (100) selon la revendication 8, dans lequel l'unité de traitement (40) est configurée pour commander la pression de brassard en continu ou par intervalles pendant des compressions en cours ou pendant des pauses dans les compressions.

10. Système (100) selon les revendications 7 à 9, dans lequel au moins un parmi l'un ou des plusieurs capteurs PPG (201, 202, 203) est configuré pour être agencé sur un côté faisant face à la peau du brassard BP (301), et dans lequel l'unité de traitement (40) est configurée pour commander une pression de brassard moyenne appliquée au patient par ledit brassard (301) pour maximiser une amplitude de l'un ou des plusieurs signaux PPG (21, 22, 23) de l'au moins un capteur PPG (201, 202, 203) et/ou une oscillation de pression à l'intérieur dudit brassard (301).

11. Système (100) selon l'une quelconque des revendications 7 à 10, comprenant en outre un ou plusieurs capteurs ECG (900, 901, 902) configurés pour mesurer un ou plusieurs signaux ECG (91), dans lequel l'unité de traitement (40) est configurée pour analyser l'un ou les plusieurs signaux ECG (91) pour faciliter l'obtention de l'une quelconque de l'une ou des plusieurs propriétés de signal PPG, et/ou comprenant en outre un capteur de compression thoracique (500) configuré pour mesurer un signal de compression thoracique (51) indiquant un ou plusieurs parmi l'impédance transthoracique, le taux de compression thoracique, la longueur des intervalles intercompressions, la force de compression thoracique, la profondeur de compression thoracique, l'accélération de compression thoracique et la vitesse de compression thoracique, dans lequel l'unité de traitement (40) est configurée pour analyser ledit signal de compression thoracique (51) et pour reconnaître et traiter ou supprimer des artéfacts dudit signal de compression thoracique (51) à partir de l'un ou des plusieurs signaux PPG (21, 22, 23) et/ou signaux ECG (91).

12. Système (100) selon l'une quelconque des revendications 7 à 11, comprenant en outre une première interface utilisateur (600) configurée pour obtenir les informations utilisateur (61) et/ou une seconde interface utilisateur (700) pour fournir les informations ROSC (71) d'une manière visible et/ou audible.

13. Procédé de prise en charge de la détection du retour de circulation spontanée pendant une réanimation cardiorespiratoire, le procédé consistant à
- obtenir un ou plusieurs signaux de photopléthysmographie, PPG (21, 22, 23) d'un patient;
- fournir un signal de commande BP (31) pour commander un capteur BP (300) pour commencer à mesurer la pression sanguine, BP, du patient;
- obtenir une ou plusieurs mesures BP (36);
- analyser une ou plusieurs propriétés de signal PPG prédéfinies de l'un ou des plusieurs signaux PPG (21, 22, 23) et générer le signal de commande BP (31) si une ou plusieurs des propriétés de signal PPG remplissent une condition prédéfinie, dans lequel l'une ou les plusieurs propriétés de signal PPG correspondent à au moins la fréquence du pouls, la force du pouls et la durée de la présence du pouls du patient, et dans lequel la condition prédéfinie comprend des seuils respectifs pour la fréquence du pouls, la force du pouls et la durée de présence du pouls, et dans lequel la condition prédéfinie sert à détecter le retour d'un pouls spontané; et évaluer un état d'un indicateur de sortie cardiogénique indiquant le retour de circulation spontanée sur la base d'une combinaison de l'un ou des plusieurs signaux PPG (21, 22, 23) avec les mesures BP.

14. Procédé selon la revendication 13, dans lequel l'une ou les plusieurs propriétés de signal PPG correspondent à une ou plusieurs parmi une durée à un niveau de fréquence du pouls prédéfini, une variation d'intervalles inter-pouls dans le temps, un certain nombre d'harmoniques induites par le coeur et une forme d'onde PPG.

15. Programme informatique comprenant des moyens de code de programme pour amener un ordinateur à exécuter les étapes du procédé selon l'une quelconque des revendications 13-14 lorsque ledit programme informatique est exécuté sur l'ordinateur.
